# EUROPEAN PATENT SPECIFICATION

(11) **EP 2 787 969 B1**
(45) Date of publication and mention of the grant of the patent: **29.09.2021**
(21) Application number: 12808607.1
(22) Date of filing: 07.12.2012
(51) Int. Cl.: A61K 9/00, A61K 9/107, A61K 47/02, A61K 47/10, A61K 47/26, A61K 47/32, A61K 47/38, A61K 47/44, A61K 31/047, A61K 31/341, A61K 31/717, A61P 27/02, A61P 27/04

(54) **EFFICIENT LIPID DELIVERY TO HUMAN TEAR FILM USING A SALT-SENSITIVE EMULSION SYSTEM**
EFFIZIENTE LIPIDABGABE AN DEN MENSCHLICHEN TRÄNENFILM UNTER VERWENDUNG EINES SALZEMPFINDLICHEN EMULSIONSSYSTEMS
UTILISATION D'UN SYSTÈME D'ÉMULSION SENSIBLE AU SEL POUR ADMINISTRER AVEC EFFICACITÉ DES LIPIDES DANS LE FILM LACRYMAL CHEZ L'HUMAIN

(30) Priority: 07.12.2011 US 201161568089 P; 17.04.2012 US 201261625401 P
(43) Date of publication of application: 15.10.2014
(73) Proprietor: ALLERGAN, INC., Irvine, CA 92612 (US)
(72) Inventor: VEHIGE, Joseph G., Laguna Niguel, California 92677 (US); SIMMONS, Peter A., Yorba Linda, California 92886 (US)
(74) Representative: Hoffmann Eitle
(86) International application number: PCT/US2012/068615
(87) International publication number: WO 2013/086449

(56) References cited:
- EP-A2- 0 028 110
- WO-A1-2013/052760
- WO-A2-2008/106228
- WO-A2-2010/141648

## Description

### CROSS-REFERENCES TO RELATED APPLICATIONS

This application claims the benefit of U.S. Provisional Application Serial Nos. 61/568,089, filed December 7, 2011 and 61/625,401 filed April 17, 2012.

### FIELD OF INVENTION

The present invention is directed to artificial tears suitable for treating dry eye syndrome and other ocular conditions in a human or other mammal.

### BACKGROUND OF THE INVENTION

Typical symptoms of keratoconjunctivitis or dry eye include feelings of dryness, burning, and a sandy-gritty eye sensation that can worsen during the day. Symptoms may also be described as itchy, scratchy, stingy or tired eyes. Other symptoms include pain, redness, a pulling sensation, and pressure behind the eye. The damage to the eye surface resulting from dry eye increases discomfort and sensitivity to bright light and both eyes usually are affected.

Because blinking coats the eye with tears, symptoms are worsened by activities in which the rate of blinking is reduced due to prolonged use of the eyes. These activities include prolonged reading, computer usage, driving or watching television. Symptoms increase in windy, dusty or smoky areas, in dry environments, high altitudes including airplanes, on days with low humidity, and in areas where an air conditioner, fan, or heater, is being used. Symptoms are less severe during cool, rainy, or foggy weather, and in humid places. Most people who have dry eyes experience mild irritation with no long-term effects. However, if the condition is left untreated or becomes severe, it can produce complications that can cause eye damage, resulting in impaired vision or possibly in the loss of vision.

Having dry eyes for a prolonged period of time can lead to tiny abrasions on the surface of the eyes. In advanced cases, the epithelium undergoes pathologic changes, namely squamous metaplasia and loss of goblet cells sometimes due to activation of T cells acting against those cells.. Some severe cases result in thickening of the corneal surface, corneal erosion, punctate keratopathy, epithelial defects, corneal ulceration, corneal neovascularization, corneal scarring, corneal thinning, and even corneal perforation. An abnormality of any one of the three layers of tears which produces an unstable tear film, may result in symptoms of keratitis sicca.

Keratoconjunctivitis sicca is usually due to inadequate tear production. The aqueous tear layer is affected, resulting in aqueous tear deficiency or lacrimal hyposecretion. The lacrimal gland does not produce sufficient tears to keep the entire conjunctiva and cornea covered by a complete layer. This usually occurs in people who are otherwise healthy. Increased age is associated with decreased tearing. This is the most common type found in postmenopausal women. Causes include idiopathic, congenital alacrima, xerophthalmia, lacrimal gland ablation, and sensory denervation. In rare cases, it may be a symptom of collagen vascular diseases, including rheumatoid arthritis, Wegener's granulomatosis, and systemic lupus erythematosus. Sjögren's syndrome and autoimmune diseases associated with Sjögren's syndrome are also conditions associated with aqueous tear deficiency. Drugs such as isotretinoin, sedatives, diuretics, tricyclic antidepressants, antihypertensives, oral contraceptives, antihistamines, nasal decongestants, beta-blockers, phenothiazines, atropine, and pain relieving opiates such as morphine can cause or worsen this condition. Infiltration of the lacrimal glands by sarcoidosis or tumors, or postradiation fibrosis of the lacrimal glands can also cause this condition.

Keratoconjunctivitis sicca can also be caused by abnormal tear composition resulting in rapid evaporation or premature destruction of the tears. When caused by rapid evaporation, it is termed evaporative dry eyes. In this condition, although the tear gland produces a sufficient amount of tears, the rate of evaporation of the tears is too rapid. There is a loss of water from the tears that results in tears that are too "salty" or hypertonic. As a result, the entire conjunctiva and cornea cannot be kept covered with a complete layer of tears during certain activities or in certain environments.

Aging is one of the most common causes of dry eyes. This is due to the fact that tear production decreases with age. It may be caused by thermal or chemical burns, or by adenoviruses. Diabetics are also at increased risk for dry eye.

An eye injury or other problem with the eyes or eyelids, such as bulging eyes or a drooping eyelid, can cause keratoconjunctivitis sicca. Disorders of the eyelid can impair the complex blinking motion required to spread tears.

About half of all people who wear contact lenses have dry eyes. This is because soft contact lenses, which float on the tear film that covers the cornea, absorb the tears in the eyes. Dry eye also occurs or gets worse after refractive surgeries, in which the corneal nerves are cut during the creation of a corneal flap, because the corneal nerves stimulate tear secretion. Dry eyes caused by these procedures usually disappear after several months.

Abnormalities of the lipid tear layer caused by blepharitis and rosacea and abnormalities of the mucin tear layer caused by vitamin A deficiency, trachoma, diphtheric keratoconjunctivitis mucocutaneous disorders and certain topical medications may cause dry eye or keratoconjunctivitis sicca.

Dry eyes can usually be diagnosed by the symptoms alone. Tests can determine both the quantity and the quality of the tears. A slit lamp examination can be performed to diagnose dry eyes and to document any damage to the eye. A Schirmer's test can measure the amount of moisture bathing the eye. This test is useful for determining the severity of the condition.

A variety of approaches can be taken to treatment, such as: avoidance of exacerbating factors, tear stimulation and supplementation, increasing tear retention, and eyelid cleansing and treatment of eye inflammation. For mild and moderate cases, supplemental lubrication is the most important part of treatment. Application of artificial tears every few hours can provide temporary relief.

Lubricating tear ointments can be used during the day, but they generally are used at bedtime due to poor vision after application. They contain white petrolatum, mineral oil, and similar lubricants. They serve as a lubricant and an emollient. Depending on the severity of the condition, ointments may be applied from every hour to just at bedtime. Ointments should not be used with contact lenses. Inflammation occurring in response to tears film hypertonicity can be suppressed by mild topical steroids or with topical immunosuppressants such as cyclosporine. WO 2010/141648 discloses an ophthalmic emulsion for use in treating dry eye syndrome comprising castor oil, olive oil, polysorbate 80, glycerin, Pemulen TR-2, carboxymethylcellulose sodium, boric acid, erythritol, carnitine, NaOH, stabilized oxochloro complex and water.

### SUMMARY OF THE INVENTION

The present invention is directed to an artificial tear emulsion of the following formulation:

**Table I**

| | | | | |
|---|---|---|---|---|
| [1] | POLYSORBATE 80 | 0.5 | % w/w | Active |
| **Grade:** NF Ph Eur | | | | |
| [2] | CARBOXYMETHYLCELLULOS E SODIUM (LOW VISCOSITY 7LFPH) | 0.5 | % w/w | Active |
| **Grade:** Ph Eur USP | | | | |
| [3] | GLYCERIN | 1.0 | % w/w | Active |
| **Grade:** Ph Eur USP | | | | |
| [4] | PURITE | 0.01 | %w/w | Preservative |
| **Grade:** | | | | |
| | BORIC ACID | 0.6 | % w/w | Buffer |
| **Grade:** NF Ph Eur | | | | |
| [5] | PEMULEN TR-2 | 0.1 | % w/w | Stabilizer |
| **Grade:** NF | | | | |
| [6] | CASTOR OIL | 0.25 | % w/w | Excipient |
| **Grade:** Eur Ph USP | | | | |
| | ERYTHRITOL | 0.25 | % w/w | Excipient |
| **Grade:** NF Ph Eur | | | | |
| | LEVOCARNITINE | 0.25 | % w/w | Excipient |
| **Grade:** Ph Eur USP | | | | |
| [7] | SODIUM HYDROXIDE | 7.3 | pH | pH Adjust |
| **Grade:** NF Ph Eur | | | | |
| [8] | WATER FOR INJECTION / PURIFIED WATER | 100 | % w/w | QS Adjust |
| **Grade:** USP | | | | |

| | | | | |
|---|---|---|---|---|
| [1] PM# 12783. Super Refined Polysorbate 80 from CRODA. Primary emulsifer and demulcent. [2] Demulcent [3] Demulcent and tonicity agent [4] Stabilized Oxychloro Complex (Purite). Add by assay value. [5] Pemulen TR-2NF (Carbomer Copolymer Type A, Tested to Ph Eur). Secondary emulsifer. [6] Lipophilic vehicle [7] pH target 7.3 [8] Hydrophilic vehicle | | | | |

The Table I formulation includes the concentrations of actives and/or excipients as disclosed above.

The formulation may be preserved or non-preserved (not containing Purite®), such as a unit dose version. This version would be the same as that in Table 1 except it would contain no Purite®.

As used herein, the term "effective amount" or "effective dose" means an amount sufficient to achieve the desired result on the process or condition, and it accordingly will depend on the ingredient and the desired result. Nonetheless, once the desired effect is known, determining the effective amount is within the skill of a person skilled in the art.

"Formulation," "composition," and "preparation" as used herein are equivalent terms referring to a composition of matter suitable for pharmaceutical use (i.e., producing a therapeutic effect as well as possessing acceptable pharmacokinetic and toxicological properties).

The term "prevent" as used herein refers to a decrease in the occurrence of dermatological symptoms (e.g., urticardial wheals) in a patient. The prevention may be complete (i.e., no detectable symptoms) or partial, so that fewer symptoms are observed than would likely occur absent treatment.

As used herein, the terms "prevent" and "treat" are not intended to be absolute terms. Treatment can refer to any delay in onset, e.g., reduction in the frequency or severity of symptoms, amelioration of symptoms, improvement in patient comfort, reduction in symptoms of dry eye, and the like. The effect of treatment can be compared to an individual or pool of individuals not receiving a given treatment, or to the same patient before, or after cessation of, treatment.

The term "therapeutically effective amount" as used herein refers to that amount of the composition or agent in a composition sufficient to ameliorate one or more aspects of the disorder.

Therapeutic efficacy can also be expressed as "-fold" increase or decrease. For example, a therapeutically effective amount can have at least a 1.2-fold, 1.5-fold, 2-fold, 5-fold, or more effect over a control.

"Treatment" as used herein includes any cure, amelioration, or prevention of a disease. Treatment may prevent the disease from occurring; inhibit the disease's spread; relieve the disease's symptoms fully or partially remove the disease's underlying cause, shorten a disease's duration, or do a combination of the above. "Treating" or "treatment" as used herein (and as well-understood in the art) also broadly includes any approach for obtaining beneficial or desired results in a subject's condition, including clinical results. Beneficial or desired clinical results can include, but are not limited to, alleviation or amelioration of one or more symptoms or conditions, diminishment of the extent of a disease, stabilizing (*i.e.,* not worsening) the state of disease, prevention of a disease's transmission or spread, delay or slowing of disease progression, amelioration or palliation of the disease state, diminishment of the reoccurrence of disease, and remission, whether partial or total and whether detectable or undetectable.

"Treating" and "treatment" as used herein may include prophylactic treatment. Treatment methods include administering to a subject a therapeutically effective amount of an active agent. The administering step may consist of a single administration or may include a series of administrations. The length of the treatment period depends on a variety of factors, such as the severity of the condition, the age of the patient, the concentration of active agent, the activity of the compositions used in the treatment, or a combination thereof. It will also be appreciated that the effective dosage of an agent used for the treatment or prophylaxis may increase or decrease over the course of a particular treatment or prophylaxis regime. Changes in dosage may result and become apparent by standard diagnostic assays known in the art. In some instances, chronic administration may be required. For example, the compositions are administered to the subject in an amount and for a duration sufficient to treat the patient.

### DETAILED DESCRIPTION OF THE INVENTION

Delivering lipids to the human tear film to supplement and enhance the native lipid layer, often deficient due to dysfunction of meibomian glands and other causes, is a recognized strategy in treating signs and symptoms of dry eye. This is in theory especially beneficial in low humidity or when other internal/external factors increase tear film evaporation. Excessive loss of water from the tear film causes an increase in salt content and causes hyperosmotic stress to the cells of the ocular surface.

The native lipid layer is very thin and the total volume of lipid is a small fraction of the total tear film volume. To enhance the structure and function of the lipid layer by topical application of a lipid-containing drop requires only a small volume of oil to be delivered; excess lipid will displace and disrupt the total aqueous volume, by far the greatest component of tears.
It is also necessary that the lipid be delivered quickly, during the brief contact time of a topical eye drop. Finally, the lipid delivered needs to become established as part of the native lipid layer, at the air interface.

The challenge of lipid release from an emulsion has been approached by using substantial amounts of lipid (1-5%) and/or building an emulsion system that readily separates. The disadvantage of this approach includes: the product requires shaking, the clarity of the emulsion is greatly reduced, the total volume of lipid delivered to the eye is potentially large and variable and tolerability can be lower than an fully aqueous eye drop.

An alternate means of lipid release involves the use of a salt-sensitive emulsion system in a product intended for topical use that is largely free of salt. This system uses a surfactant and viscosity-increasing polymer to hold the lipid (eg. castor oil) in a stable sub-micron emulsion. When mixed with human tear, the natural salt content (often further elevated in dry eye) is sufficient to rapidly cause a drop in product viscosity due to action on the polymer structure. This loss of viscosity allows lipid release to occur to a significantly greater degree and much faster.

Efficiency of lipid delivery can be defined as the amount of lipid released from the emulsion, as a proportion of total lipid content, over time under standard test conditions.

Efficiency of lipid delivery in the presence of salt is supported, for example, using simple laboratory methods. Specifically, when diluted with water, this system shows a loss of viscosity proportional to water volume added. When exposed to salt (NaCl) by mixing 1:1 with even a weak saline solution (30 mOsm) a loss of viscosity of over 60 % occurs vs. 50% when mixed with water. Higher saline strength (up to about 600 mOsm) caused significantly greater loss of viscosity, confirming action of salt on polymer structure.

The release of lipid was demonstrated using a controlled centrifuge with real-time integrated optical detector (Lumisizer). During 2 minutes of 4000 RPM stress, uniformity of the emulsion was confirmed by equivalent optical transmission from bottom to top of the centrifuge sample holder for both full strength and water-diluted product. However, diluting the product with saline (volume and concentration replicating on-eye use) showed a clear and remarkable change in product uniformity consistent with lipid release and migration to the top of the sample holder, consistent with "floating" to the air interface. Surprisingly and beneficially, this may occur without coalescence (no increase in average lipid droplet size) allowing the lipid to mix into the native layer more effectively. Average particle (lipid droplet size) was unchanged when saline was added (Horiba)

Clinical results have confirmed that the new lipid emulsion system works effectively in prolonging TBUT (tear break-up time) yet demonstrates tolerability and comfort improvements vs. an emulsion more optimized for drug delivery.

The benefits of using a salt-sensitive emulsion system as shown in Table I, that is largely free of salts, include but are not limited to:
1) No need to shake the product-excellent in bottle stability and uniformity;
2) Efficient delivery of lipid on eye due to the salt-induced decrease in viscosity and destabilization of emulsion structure enabling more efficient lipid release;
3) Improved tolerability by lowering total lipid content;
4) Effective stabilization and supplementation of the native lipid layer; and
5) Possibly greater delivery of beneficial lipid in patients with higher tear salt content, a so-called "smart" vehicle.

The incorporation of osmoprotectants (1-carnitine and erythritol) and humectants/lubricants (glycerin and carboxymethylcellulose increases the clinical usefulness of this product to a broader range of dry eye patients than other emulsion systems targeting lipid deficiency or meibomian gland dysfunction.

Example 1 - A Multicenter, Investigator-masked, Randomized, 4-Arm, Parallel-group Study to Evaluate the Safety, Efficacy, and Acceptability of a Unit-dose Eye Drop Formulation in Subjects With Dry Eye Disease

The objective of the study was to evaluate the safety, efficacy, and acceptability of the formulation of Table 1, but without containing Purite®, referred to as a Next Generation Emulsion Unit-dose or ("NGE UD") in subjects with signs and symptoms of dry eye disease.

### Methodology

This was a multicenter, investigator-masked, randomized, active-controlled, 4-arm, parallel group study designed to compare the safety, efficacy, and acceptability of NGE UD to commercially available OPTIVE™ Sensitive Preservative-free Lubricant Eye Drops Unit-dose ("OPTIVE UD"), NGE UD to Next Generation Emulsion Multidose ("NGE MD") (same formulation as Table 1 but with Purite®), and NGE MD to OPTIVE™ Lubricant Eye Drops Multidose ("OPTIVE MD").

The planned study duration was 30 days for each subject and consisted of up to 3 scheduled visits (days 1 [baseline], 7, and 30 [exit]). On day 1, eligible subjects with signs and symptoms of dry eye disease were assigned according to a 2:2:1:1 treatment allocation ratio to use NGE UD, OPTIVE UD, NGE MD, or OPTIVE MD, respectively. The study randomization was stratified by baseline Ocular Surface Disease Index© (OSDI) score (mild/moderate symptoms = score of 18 to 32; severe symptoms = score of > 32 to 65). Approximately 300 subjects were to be enrolled at 13 to 14 sites within the USA in order to have 288 completed subjects assuming a dropout rate of approximately 5%. Subjects were instructed to instill 1 to 2 drops of their assigned study product in each eye, as needed, but at least 2 times daily for 30 days.

### Number of Subjects (Planned and Enrolled)

Approximately 300 subjects were planned to be enrolled in this study. A total of 315 subjects were enrolled.

### Diagnosis and Main Criteria for Eligibility

### Diagnosis/Subjects with signs and symptoms of dry eye disease

### Key Inclusion Criteria:

Male or female subjects, at least 18 years of age, with a baseline (day 1) OSDI score of ≥ 18 and ≤ 65 (based on a 0 to 100 scale) were eligible for enrollment. Subjects must have been using topical ophthalmic drops for dry eye at least twice daily, for at least 3 months prior to baseline, on average. If there was daily use of RESTASIS® Cyclosporine Ophthalmic Emulsion, it must have been in use for ≥ 6 months. Three consecutive tear break-up time (TBUT) tests ≤ 10 seconds in at least 1 eye at baseline were required. Using the modified National Eye Institute (NEI) Grid, all subjects had to have at least a Grade 1 staining in at least 1 of the 5 zones of the cornea or in at least 1 of the 6 zones of the conjunctiva that is related to dry eye in at least 1 eye at baseline.

### Key Exclusion Criteria:

Key exclusion criteria included a Schirmer test (with anesthesia) ≤ 2 mm in either eye at baseline; corneal or conjunctival staining score of 5 (modified NEI Grid) at baseline in any of the 5 corneal or 6 conjunctival zones of either eye; use of systemic medications that could have affected a dry eye condition or vision, unless that medication had been used at the same dose for at least 3 months prior to study enrollment and the dosage was not expected to change during the course of the study; history of anterior segment surgery or trauma that could have affected corneal sensitivity (eg, cataract surgery, laser-assisted in situ keratomileusis [LASIK], photorefractive keratectomy, or any surgery involving a limbal or corneal incision) within 12 months prior to baseline; and current use of, and/or use within 2 weeks prior to baseline, and/or likely use during the study period of any topical ophthalmic medications (eg, topical ophthalmic steroids, glaucoma drops, any topical cyclosporine product other than Restasis®. Subjects who discontinued use of daily Restasis® less than 3 months prior to baseline were excluded from the study.

**Duration of Treatment:** The total duration of exposure to the study product (drops) for each subject was 30 days. The visit schedule consisted of a baseline visit (day 1) and 2 follow-up visits on days 7 (± 3 days) and 30/early exit (± 7 days).

### Efficacy and Safety Measurements

### Efficacy: Primary - OSDI questionnaire score

Secondary - TBUT (with fluorescein), corneal staining (modified NEI Grid, with fluorescein), conjunctival staining(modified NEI Grid, with lissamine green), and Schirmer test (with anesthesia)

Other - Acceptability Questionnaire and Study Product Usage Questionnaire

### Safety:

The safety measures were adverse events, biomicroscopy, and distance visual acuity.

### Statistical Methods:

The intent-to-treat (ITT) population consisted of all randomized subjects and was used for analyses of efficacy data based on the treatment randomized. The safety population consisted of all treated subjects and was used for analyses of all safety data based on the actual treatment received. The per-protocol (PP) population consisted of randomized subjects who had no major protocol violations, as determined prior to database lock.

The primary efficacy variable was the change from baseline in OSDI score at day 30 in the ITT population. The primary efficacy analysis was performed on the change from baseline in OSDI score at day 30 via a 2-way analysis of variance (ANOVA) model with treatment and baseline OSDI stratification as the main effects.

Last observation carried forward (LOCF) was used to impute missing data. Noninferiority was tested using a 2-sided confidence interval (CI). The treatment difference and 95% CI in change from baseline in OSDI score at day 30 between NGE UD and OPTIVE UD (NGE UD minus OPTIVE UD) were calculated based on the ANOVA model. Non-inferiority was established if the upper limit of the 95% CI was less than the prespecified margin of 7.3.

The Secondary efficacy measures included TBUT, corneal staining, conjunctival staining, and Schirmer test. The raw values of these measures were summarized for the ITT population, with missing data imputation using LOCF at each scheduled follow-up visit. The treatment difference and 95% CI for between-treatment comparisons were calculated. The treatment differences and 95% CIs in change from baseline in OSDI score at day 30 between NGE UD and NGE MD, NGE MD and OPTIVE MD were also analyzed as secondary efficacy variables.

Acceptability was measured using the Acceptability Questionnaire, and product usage was measured using the Study Product Usage Questionnaire. Comparisons across groups were performed using ANOVA model with treatment and baseline OSDI stratification as the main effects.

The safety variables included adverse events, biomicroscopy, and distance visual acuity. Since both eyes were treated, both eyes were included in the safety analyses. The Medical Dictionary for Regulatory Activities (MedDRA) nomenclature was used to code adverse events. The number and percent of subjects with clinically significant biomicroscopy findings at one or more visits in either eye were tabulated. The overall frequency distribution was analyzed using Pearson's chi-square test. For a clinically significant biomicroscopic finding (more than 1 severity grade increase [worsening] from baseline) with an incidence rate of ≥ 5% in any treatment group, the mean severity grade and the frequency distribution of severity scores were summarized at each scheduled visit.

Data from the eye with the worst severity at the scheduled visit was tabulated. For distance visual acuity data, the total numbers of letters read correctly were summarized based on the eye with worse change from baseline at each scheduled visit. The frequency distribution was analyzed using Pearson's chi-square test.

A total of 315 subjects were enrolled in the study and included in the ITT population; 105 subjects in the NGE UD group, 103 subjects in the OPTIVE UD group, 51 subjects in the NGE MD group, and 56 subjects in the OPTIVE MD group. Overall, 310 (98.4%) subjects in the ITT population completed the study. Of the subjects included in the per protocol population, 99.3% (303/305) completed the study whereas 98.4% (310/315) of the subjects in the safety population completed the study. A total of 384 subjects were screened of which 69 subjects were screen failures.

In the ITT population, the mean age of all subjects was 54.8 years (standard deviation 14.33) with 83.2% (262/315) of subjects in the > 40 years age group. In addition, 81.0% (255/315) of all subjects were female and 84.4% (266/315) were Caucasian.

### Efficacy:

- The primary efficacy endpoint was met. At day 30, no statistically significant difference was observed between the NGE UD and the OPTIVE UD groups in the mean change from baseline in OSDI score (95% confidence interval [-5.42, 2.51]), in the ITT population. The NGE UD formulation was noninferior to the OPTIVE UD formulation in reducing the severity of symptoms of dryness as measured by the change from baseline in OSDI score.
- Similar to the ITT population, there was no statistically significant difference between the NGE UD and OPTIVE UD groups of the PP population in the mean change from baseline in OSDI score at day 30. The 95% confidence interval at the day 30 visit was (-5.72, 2.37); with an upper limit that is lower than the clinically relevant margin of 7.3.
- In all 4 treatment groups, there was a statistically significant difference (p < 0.001) in the mean change from baseline in OSDI score at the day 7 and day 30 visits for both the ITT and the PP population.
- The NGE UD group was noninferior to the NGE MD group in the mean change from baseline in OSDI score at day 30.
- The NGE UD group was noninferior to the OPTIVE UD and NGE MD groups in the secondary efficacy measures of TBUT, corneal staining, conjunctival staining, and Schirmer test.
- Overall, there were no statistically significant differences between the NGE UD and OPTIVE UD groups, NGE UD and NGE MD groups, or NGE MD and OPTIVE MD groups, in the mean values for each question of the acceptability questionnaire at the day 7 and day 30 visits (except for question 5 in the NGE MD versus OPTIVE MD comparison at day 7 and NGE UD versus NGE MD comparison at day 30), and in the mean number of times per day that the study product was used during the week prior to the day 7 and day 30 visits.

### Safety:

- At least 1 treatment-emergent adverse event (TEAE) of any causality was reported in 11.4%, 15.5%, 13.7%,and 10.7% of subjects in the NGE UD, OPTIVE UD, NGE MD and OPTIVE MD groups, respectively.
- No deaths were reported in the study. Two serious adverse events were reported (bile duct stone [NGE UD group] and ankle fracture [OPTIVE MD group]), none of which were treatment related in the opinion of the investigator

Overall 3 subjects discontinued from the study due to adverse events, 1 subject each in the NGE UD, NGE MD and OPTIVE MD groups.
- Treatment-related TEAE were reported in 4.8%, 8.7%, 7.8%, and 5.4% of subjects in the NGE UD, OPTIVE UD, NGE MD, and OPTIVE MD groups, respectively. The most common treatment-related adverse events (preferred terms) across treatment groups were instillation site pain and vision blurred; NGE UD (3.8%, 2.9%), OPTIVE UD (3.9%, 2.9%), NGE MD (3.9%, 0.0%), and OPTIVE MD (3.6%, 1.8%).
- In the majority of the subjects, no change was observed in the distance visual acuity at day 30 for all 4 treatment groups.

### Conclusions

Efficacy: The results of this study demonstrate that the NGE UD formulation is noninferior to the OPTIVE UD formulation in reducing the severity of symptoms of dryness in subjects with mild to severe dry eye.

Safety: NGE UD appeared to be well tolerated during the study. The most commonly reported treatment-related adverse events were instillation site pain and vision blurred. Throughout the study, there were no treatment-related serious adverse events. The safety profile was consistent with OPTIVE UD, OPTIVE MD, and NGE MD. This is supportive of the safety of the NGE UD formulation in clinical use, and confirms the safety of the NGE MD formulation.

## Claims

1. An artificial tear emulsion, wherein said emulsion is a sub-micron emulsion, wherein said emulsion is of the following formulation:
0.5% w/w Polysorbate 80,
0.5% w/w carboxymethylcellulose sodium,
1.0% w/w glycerin,
0.6% w/w boric acid,
0.1% w/w pemulen, wherein Pemulen is a carbomer copolymer type A,
0.25% w/w castor oil,
0.25% w/w erythritol,
0.25% w/w levocarnitine,
sodium hydroxide to adjust the pH to 7.3,
water;
and optionally 0.01% w/w Purite®, wherein Purite® is a stabilized oxychloro complex.

2. The emulsion of claim 1 for use in a method of treating dry eye.

3. The emulsion of claim 1 or the emulsion for use of claim 2 wherein the composition is preservative free.

## Patentansprüche

1. Künstliche Tränenemulsion, worin die Emulsion eine Submikronemulsion ist, worin die Emulsion die folgende Formulierung aufweist:
0,5 Gew.-% Polysorbat 80,
0,5 Gew.-% Carboxymethylcellulose-Natrium,
1,0 Gew.-% Glycerin,
0,6 Gew.-% Borsäure,
0,1 Gew.-% Pemulen, worin Pemulen ein Carbomer Copolymer vom Typ A ist,
0,25 Gew.-% Rizinusöl,
0,25 Gew.-% Erythrit,
0,25 Gew.-% Levocarnitin,
Natriumhydroxid, um den pH-Wert auf 7,3 einzustellen, Wasser,
und wahlweise 0,01 Gew.-% Purite®, worin Purite® ein stabilisierter Oxychloro-Komplex ist.

2. Emulsion gemäß Anspruch 1 zur Verwendung in einem Verfahren zur Behandlung von trockenem Auge.

3. Emulsion gemäß Anspruch 1 oder Emulsion zur Verwendung gemäß Anspruch 2, worin die Zusammensetzung frei von Konservierungsmitteln ist.

## Revendications

1. Émulsion de larmes artificielles, dans laquelle ladite émulsion est une émulsion submicronique, dans laquelle ladite émulsion est de la formulation suivante :
0,5 % en p/p de polysorbate 80,
0,5 % en p/p de carboxyméthylcellulose sodique,
1,0 % en p/p de glycérine,
0,6 % en p/p d'acide borique,
0,1 % en p/p de pemulen, dans laquelle le Pemulen est un copolymère de carbomère de type A,
0,25 % en p/p d'huile de ricin,
0,25 % en p/p d'érythritol,
0,25 % en p/p de lévocarnitine,
de l'hydroxyde de sodium pour ajuster le pH à 7,3,
de l'eau ;
et, facultativement, 0,01% en p/p de Purite®, dans laquelle le Purite® est un complexe d'oxychloro stabilisé.

2. Émulsion selon la revendication 1 destinée à être utilisée dans un procédé de traitement de l'œil sec.

3. Émulsion selon la revendication 1 ou émulsion destinée à être utilisée selon la revendication 2, dans laquelle la composition est sans conservateur.
